# DEMANDE DE BREVET EUROPEEN

(11) **EP 2 818 158 A1**
(43) Date de publication de la demande: **31.12.2014**
(21) Numéro de dépôt: 14173466.5
(22) Date de dépôt: 23.06.2014
(51) Int. Cl.: A61K 8/64, A61Q 19/08, A61K 8/97

(54) **Compositions cosmétiques comprenant des extraits de plantes pour lutter contre le vieillissement cutané**

(30) Priorité: 24.06.2013 FR 1356013
(71) Demandeur: CASTER, 75008 Paris (FR)
(72) Inventeur: Choulot, Jean-Christophe, 78120 RAMBOUILLET (FR); Hajem, Neïla, 78290 CROISSY SUR SEINE (FR)
(74) Mandataire: Casalonga

(57) **Abrégé**

La présente invention a pour objet une composition cosmétique contenant au moins un extrait d'arbre à soie, un extrait de grenadier et un peptide de séquence SEQ ID N°1. L'invention a également pour objet un procédé cosmétique pour lutter contre le vieillissement et/ou le flétrissement de la peau, comprenant l'application topique de la composition sur la peau.

<110>  CASTER

<120>  Compositions cosmétiques comprenant des extraits de plantes pour
lutter contre le vieillissement cutané

<130>  B13-2686EP GD/LM

<150>  FR1356013

<151>  2013-06-24

<160>  1

<170>  PatentIn version 3.1

<210>  1
<211>  4
<212>  PRT
<213>  artificial sequence

<220>
<223>  synthetic

<220>
<221>  MISC_FEATURE
<222>  (4)..(4)
<223>  ureido citrulline


<400>  1

Lys Asp Val Xaa
1

## Description

La présente invention a pour objet une composition cosmétique contenant au moins un extrait d'arbre à soie, un extrait de grenadier et un peptide de séquence SEQ ID N°1. L'invention a également pour objet un procédé cosmétique pour lutter contre le vieillissement et/ou le flétrissement de la peau, comprenant l'application topique de la composition sur la peau.

Les Multidrug Résistance Proteins (MDR) sont des protéines transmembranaires présentes à la périphérie des cellules, ces protéines jouent le rôle de «pompes» capables d'excréter les substances toxiques hors de la cellule.

Sur les cellules cancéreuses, le nombre de MDR est particulièrement important. Dans ces cellules, le rôle des MDR semble être de leur permettre de rejeter les médicaments de chimiothérapie, perçus comme des substances toxiques : les MDR permettent aux cellules cancéreuses de résister aux drogues et empêchent leur élimination. Ce mécanisme pourrait ainsi expliquer pourquoi les cellules cancéreuses sont apparemment immortelles.

Les MDR sont aussi responsables de la résistance de bactéries à des antibiotiques.

Sur les cellules saines, les protéines MDR éliminent les toxines de toute nature, y compris les molécules issues du phénomène de glycation comme les Advanced Glycosylation End products (AGE), par une expulsion active des composés toxiques endogènes hors de la cellule.

La longévité, ainsi que le nombre de divisions de la cellule, sont liés à la quantité et la fonctionnalité des MDR, qui perdent la plupart de leur fonction à la fin de la vie reproductive : ces protéines déterminent donc la durée de vie des cellules. Un lien est d'ailleurs établi entre la voie des MDR et celle de la protéine SIRT1, surnommée protéine de longévité.

Une étude sur le sujet des MDR a été réalisée sur des levures, qui représentent un modèle classique pour l'étude du vieillissement (Eldakak et al., Nature cell biology, august 2010, vol 12 (8)799-807). La particularité des levures est leur prolifération par une division asymétrique au cours de laquelle une cellule dite mère va engendrer une cellule dite fille. Au cours de cette division, la cellule mère conserve l'ensemble des protéines endommagées et autres composés cellulaires qui pourraient être nocifs, préservant ainsi la cellule fille. De ce fait, avec le vieillissement, la cellule accumule des toxines intracellulaires. Lors de la division asymétrique, la répartition des MDR se fait de manière inégale :
- la cellule mère conserve les protéines MDR originales vieillies,
- tandis que la fille reçoit les protéines MDR nouvellement formées. Cette dernière possède donc une meilleure capacité de détoxication.

Si une levure est mutée par suppression du gène codant pour une MDR, alors on note une réduction de 11 à 66% du nombre de cellules filles ; ce qui traduit une diminution de la prolifération cellulaire. Si au contraire les levures sont mutées et comportent une copie supplémentaire de chaque gène codant pour la protéine MDR, c'est une augmentation de 10 à 20% du nombre de divisions des cellules qui est observée. Sur les cellules de levure dont la division est asymétrique, la présence de MDR en plus grande proportion permet d'augmenter leur longévité. Ainsi, le lien entre longévité cellulaire d'une colonie de levures et rôle des MDR est ainsi établi.

Dans l'organisme des mammifères, la seule division asymétrique qui se fait est celle des cellules souches. Il est fort probable que, comme pour les levures, une augmentation de l'expression des MDR sur les cellules souches permettra d'augmenter leur capacité proliférative, améliorant d'autant la longévité du tissu concerné, C'est particulièrement vrai pour l'épiderme. En effet, contrairement au derme, l'épiderme contient un nombre important de cellules souches situées au niveau de la lame basale.

Au cours du vieillissement, les protéines MDR sont de moins en moins fonctionnelles : les cellules accumulent de plus en plus de toxines intracellulaires qu'il sera de plus en plus difficile d'éliminer.

Ainsi, stimuler l'activité des MDR permet de conserver la capacité des cellules à éliminer les composés toxiques endogènes, améliorer la capacité de détoxication des cellules, assurer une régénération cellulaire, et de ce fait, ralentir les phénomènes associés au vieillissement:

La demanderesse a mis au point de nouvelles compositions cosmétiques, qui constituent l'objet de l'invention.

L'invention a également pour objet un procédé cosmétique pour lutter contre les effets du vieillissement et/ou flétrissement de la peau, comprenant l'application sur la peau d'une composition telle que définie précédemment.

D'autres objets apparaîtront à la lecture de la description et des exemples qui suivent.

Les compositions conformes à l'invention comportent dans un milieu physiologiquement acceptable, au moins un extrait d'arbre à soie, un extrait de grenadier et un peptide de séquence SEQ ID N°1.

L'extrait d'arbre à soie utilisé provient *d'Albizia Julibrissin.* Cet arbre, appelé Arbre à soie ou Mimosa de Constantinople, est un arbre à feuillage caduc de la famille des Mimosaceae. Il est originaire d'Asie de l'Est et du Sud, mais il a été répandu par l'homme sur presque tous les continents.

Des extraits *d'Albizia Julibrissin* ont déjà été proposés en cosmétique. Le document JP2009242296 décrit un extrait *d'Albizia Julibrissin* capable d'une action inhibitrice de la production de mélanine et stimulante de la production de collagène. Le document KR20100090530 décrit l'activité inhibitrice des métallo-protéinases d'un extrait de cortex *d'Albizia Julibrissin.* Le document KR20020080657 décrit une activité anti-radicalaire d'un extrait d'écorces d'*Albizia Julibrissin.* Le document JP4342519 décrit l'utilisation d'un extrait d'*Albizia Julibrissin* pour inhiber l'activité de la tyrosinase. Le document JP2000143488 décrit les propriétés humectantes pour la peau et les cheveux d'un extrait *d'Albizia Julibrissin.* Le document JP2048515 décrit l'utilisation d'un extrait d'*Albizia Julibrissin* dans un produit capillaire pour prévenir la perte des cheveux et favoriser leur croissance. Cette plante possède des propriétés protectrices démontrées (glycation, la lipofushine, renforcement de l'activité de la glyoxalase et du protéasome, protection et réparation du réseau microvasculaire).

De préférence, l'écorce de cet arbre est utilisée.

Le mode d'obtention de cet extrait est de préférence le suivant : La poudre d'écorce d'*Albizia Julibrissin* provient de chez PMA 28 ou de l'Herboristerie Caillaud. Cette poudre d'écorce d'*Albizia Julibrissin* subit une extraction aqueuse à chaud sous reflux. L'extrait obtenu est ensuite mélangé à de la glycérine. Ainsi, de préférence l'extrait d'arbre à soie est ainsi de préférence un extrait hydroalcoolique.

Dans les compositions de l'invention, la concentration finale d'extrait sec d'arbre à soie est de 0.1% à 10% en poids de la composition totale.

Le Grenadier porte le nom latin scientifique de *Punica granatum.* C'est un arbuste de 2 à 5 m de haut qui appartient à la famille botanique des Punicaceae. Son origine est probablement la Perse. Le tronc, recouvert d'une mince écorce grisâtre, se ramifie irrégulièrement en branches plus ou moins épineuses portant des feuilles opposées à court pétiole, ovales, entières, vert foncé, luisantes, sans stipule. Les fleurs rouges, pourpres ou grenats sont solitaires à l'aisselle des feuilles ou réunies par groupe de 2 ou 3 (G., Bezanger-Beauquesne L., Debraux G., Ressources Médicinales de la Flore Française, Vigot Frères Editeurs, 1961, p. 838-842).

Les parties utilisées du grenadier sont l'écorce des racines et celle du fruit, les fleurs et le suc du fruit. On trouve ces différentes parties décrites dans les anciennes pharmacopées espagnole, américaine et française. De préférence, on utilise la fleur de grenadier. L'extrait de fleur de grenadier, contenant des composés antioxydants, prévient la cytotoxicité liée aux UVs en réduisant la synthèse des MMP-1, et ainsi rétablissant la vitalité cellulaire après irradiation.

Le procédé d'extraction est le suivant:

Les fleurs séchées de grenadier provenant de chez PMA 28 ou de l'Herboristerie Caillaud sont broyées puis extraites dans l'éthanol sous agitation à température ambiante sous reflux.

Une fois l'extraction réalisée, la solution est filtrée puis concentrée et séchée afin d'obtenir une poudre. L'extrait de fleur de grenadier est ainsi de préférence un extrait hydroalcoolique.

Dans les compositions de l'invention, la concentration finale d'extrait de fleur de grenadier est de 0.1% à 10% en poids de la composition totale.

Le peptide utilisé est le tripeptide-9 citrulline de séquence H-Lys-Asp-Val-Cit-NH₂ (SEQ ID N°1). Celui-ci est un peptide synthétique obtenu par réaction de lysine et valine avec l'acide 5-ureido-2-amino pentanoique (Lipotec, Espagne). Ce peptide a la capacité de protéger l'ADN cellulaire de l'oxydation et de la glycation.

De préférence, ce peptide est utilisé à une concentration de 0.01% à 10 en poids de la composition totale.

La composition selon l'invention est adaptée à une application topique sur la peau et comprend donc généralement un milieu physiologiquement acceptable, c'est-à-dire compatible avec la peau. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut comprendre avantageusement une phase aqueuse. La composition peut comprendre de l'eau en une teneur allant de 1 à 99% en poids par rapport au poids total de la composition, de préférence allant de 40 à 75% en poids par rapport au poids total de la composition.

La composition selon l'invention peut se présenter sous toutes les formes galéniques normalement utilisées pour une application topique sur la peau, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick.

Avantageusement, la composition selon l'invention est sous forme d'une émulsion huile dans eau ou d'un gel, une crème ou un stick.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les coémulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40, le tristéarate de sorbitane, les stéarates de sorbitane oxyéthylénés disponibles sous les dénominations commerciales Tween 20 ou Tween 60, par exemple et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition définie précédemment est utilisée en application topique dans le cadre d'un procédé cosmétique pour lutter contre le vieillissement et/ou le flétrissement de la peau.

L'invention sera maintenant illustrée à l'aide des exemples non limitatifs suivants.

### Exemple 1 : Evaluation de la cytotoxicité de la composition selon l'invention

Ce test sert à définir la dose maximum tolérable que l'on peut utiliser sur les cellules sans effet toxique, par une quantification rapide et sensible de la prolifération et de la viabilité cellulaires en fonction des compositions selon l'invention.

Brièvement, le test colorimétrique est basé sur l'activité d'une enzyme mitochondriale, la succinate déshydrogénase, qui dégrade le substrat MTT jaune (3-[4,5-diméthylthiazol-2yl]-2,5-diphényltétrazolium bromide) en cristaux de formazan (violet). La quantité de sel formazan produite par les cellules à partir du MTT indique la vitalité cellulaire et se mesure par spectrophotométrie à 570 nm.

Les cellules à tester sont des cellules primaires humaines de fibroblastes (HDFa) (Tebu-Bio - Cat 106-05a ; lot 2138 - prélevées sur plastie faciale d'une femme caucasienne de 42 ans. Au troisième passage, les cellules ont été ensemencées dans des plaques 96 puits à raison de 2000cellules dans un volume de 200µL de milieu de culture (Tebu-Bio - Fibroblast Growth Medium - 500 ml - Réf. 95116500).

Ces cellules sont ensuite incubées 24h à 37°C et 5% de CO2 pour permettre l'adhésion à la plaque. Au 7^{ème} jour après ensemencement des cellules (le milieu ayant été remplacé par du milieu frais tous les deux jours), on retire le milieu de culture (Tebu-Bio - Fibroblast Growth Medium - 500 ml - Réf. 95116500)et on ajoute 200µL de l'actif à tester dilué dans du milieu de culture (pas moins de 50% de milieu), puis on remet à incuber (37C, 5% CO2).

Les compositions sont préparées de la sorte :
Pour l'extrait d'arbre à soie, les concentrations utilisées sont 0.005 %, 0.002 %, 0.001 %, 0.0008 %, 0.0006 %, 0.0004 %, 0.0002 %, 0.0001 %, et 0.00001 %.
Pour le peptide de séquence SEQ ID N°1 utilisé, les concentrations sont 0.005 %, 0.0002 %, 0.0001 %, 0.00008 %, 0.00006 %, 0.00004 %, 0.00002 %, 0.00001 %, et 0.000001 %.
Pour l'extrait de fleur de grenade, les concentrations utilisées sont de 2 %, 1 %, 0.8 %, 0.6 %, 0.4 %, 0.2 %, 0.1 %, 0.05 %, et 0.01 %.

Le jour du test, on prépare 2 mL de solution fraîche de MTT en diluant la poudre de MTT (Thiazolyl Blue Tetrazolium Bromide, Sigma ref. M 5655) à 5mg/ml dans du PBS.

On ajoute 20µL de solution de MTT à chaque puits. Après homogénéisation, on remet la plaque à incuber (37C, 5% CO2) pendant 3 à 4 heures pour permettre la métabolisation du MTT.

Pendant ce temps, on prépare la solution de solubilisation du MTT (DMSO/Isopropanol - 1:1). On retire le milieu de culture par aspiration, puis on sèche la plaque avec du papier absorbant. On re-suspend ensuite les cristaux de formazan qui se sont formés avec 150µL de solution de solubilisation du MTT, par agitation 150rpm pendant 15 minutes à température ambiante.

La densité optique est ensuite lue à 570nm au spectrophotomètre Perkin Elmer, Enspire® Multimode Reader, en retirant la DO à 670nm (bruit de fond).

Le résultat s'exprime en fonction du % d'absorption par rapport aux % d'absorption relevé pour les cellules contrôles (non traitées par un composé).

Les résultats pour les différents produits testés sont présentés dans le tableau ci-dessous (% de vitalité cellulaire par rapport aux cellules non traitées, dont la vitalité est de 100%):

| | | | | | |
|---|---|---|---|---|---|
| Concentration Extrait d'arbre à soie (en %) | 0,005 | 0,002 | 0,001 | 0,0008 | 0,0006 |
| % vitalité | 75,9 | 96,7 | 82,8 | 89,6 | 101,0 |

| | | | | |
|---|---|---|---|---|
| Concentration Extrait d'arbre à soie (en %) | 0,0004 | 0,0002 | 0,0001 | 0,00001 |
| % vitalité | 107,7 | 92,0 | 102,9 | 101,1 |

Pour le peptide

| | | | | | |
|---|---|---|---|---|---|
| Concentration Peptide (en %) | 0,005 | 0,0002 | 0,0001 | 0,00008 | 0,00006 |
| % vitalité | 49,7 | 47,6 | 39,8 | 57,3 | 44,7 |

| | | | | |
|---|---|---|---|---|
| Concentration Peptide (en %) | 0,00004 | 0,00002 | 0,00001 | 0,000001 |
| % vitalité | 72,0 | 66,9 | 71,0 | 94,6 |

Pour l'extrait de fleur de grenade

| | | | | | |
|---|---|---|---|---|---|
| Concentration Extrait de fleur de grenade (en %) | 2 | 1 | 0,8 | 0,6 | 0,4 |
| % vitalité | 57,1 | 74,0 | 79,5 | 87,9 | 94,6 |

| | | | | |
|---|---|---|---|---|
| Concentration Extrait de fleur de grenade (en %) | 0,2 | 0,1 | 0,05 | 0,01 |
| % vitalité | 99,2 | 110,8 | 117,3 | 126,0 |

Une dernière série de test a été faite, avec les solutions S₀ à S₈ correspondant à des dilutions successives d'une solution S₀ contenant 0,001% d'extrait d'arbre à soie, 4.10⁻⁵ % de peptide et 0,2% d'extrait de fleur de grenadier.

| S₀ | S₁ | S₂ | S₃ | S₄ |
|---|---|---|---|---|
| | S₀/1,33 | S₀/1,5 | S₀/2 | S₀/5 |

| S₅ | S₆ | S₇ | S₈ |
|---|---|---|---|
| S₀/10 | S₀/20 | S₀/50 | S₀/100 |

Dans ces conditions, les résultats sont les suivants.

| Solution s Mélange | S₀ | S₁ | S₂ | S₃ | S₄ |
|---|---|---|---|---|---|
| % vitalité | 79,9 | 81,2 | 88,2 | 85,3 | 88,2 |

| Solutions Mélange | S₅ | S₆ | S₇ | S₈ |
|---|---|---|---|---|
| % vitalité | 58,2 | 98,5 | 96,5 | 109,5 |

D'après ces résultats, la concentration S2, qui permet de conserver près de 90 % de viabilité cellulaire est choisie pour le reste des essais. Elle correspond à des concentrations de :

| Extrait d'arbre à soie | Peptide | Extrait de fleur de grenade |
|---|---|---|
| 6,6.10⁻⁴% | 2,6.10⁻⁵% | 0,13% |

### Exemple 2 : Test d'activité des MDR

Les MDR sont des protéines de transport permettant d'expulser hors de la cellule des molécules « indésirables », ce qui permet de détoxifier la cellule. En testant l'activité cellulaire MDR après traitement par des actifs, on pourra en déduire la capacité des actifs à augmenter le pouvoir détoxifiant cellulaire.

Dans les cellules normales, la calcéine non fluorescente pénètre dans la cellule et s'y accumule. Elle y est hydrolysée par les estérases endogènes, et devient fluorescente. Dans les cellules dont l'expression des MDR est augmentée, la présence de ces transporteurs de protéines permet d'expulser la calcéine hors de la cellule avant qu'elle ne soit clivée : il n'y a pas de fluorescence.

Pour ce faire, on utilise le Kit Molecular Probes Vybrant MDR Résistance Assay Kit (V13180 Invitrogen life Science).

Ainsi, dans ce test, plus la fluorescence est faible, plus les MDR sont actives.

A J0, on prépare autant de plaques 96 puits noirs à fond transparent traitées pour la culture des cellules (Corning) que nécessaire avec 7000 cellules HDFa/ puits (cellules primaires humaines de fibroblastes (Tebu-Bio - Cat 106-05a ; lot 2138 - prélevées sur plastie faciale d'une femme caucasienne de 42 ans) dans un volume de milieu de culture de 150µL/puits. Ces cellules sont alors au 4ème passage. On laisse les cellules reposer 48h pour adhésion dans une étuve à 37°C et 5% de CO2. A J2, on supprime le milieu de culture et on le remplace par 150µL / puits d'actif dilué dans du milieu de culture en prévoyant 5 puits pour chaque concentration d'actif, et notamment :
- 5 puits avec PBS (qui ne sera pas traité par calcéine, blanc)
- 5 puits avec PBS (qui sera traité par calcéine, contrôle)
- 5 puits avec du Vérapamil à 30 µg/mL final en puits (contrôle négatif). Pour cela, on ajoute 1mL d'éthanol absolu à l'ampoule de 10mg de verapamil (composant C du kit V13180 Invitrogen life Science) et on agite vigoureusement jusque totale dissolution. Cette solution mère à 10mg/mL est ensuite diluée dans du PBS jusqu'à obtention de la concentration d'utilisation.
- 5 puits avec FGF (Sigma) à 5nM final en puits (contrôle positif) ou KGF (Sigma) à 25nG/mL.

On incube les cellules comprenant des actifs à 37°C, 5% CO2 pendant 24heures.

A J3, on prépare une solution de marquage, en diluant dans du PBS la solution de calcéine du kit à 1mM (Composant A du kit V13180 Invitrogen life Science) pour atteindre une concentration de 1µM (dilution 1000x) on prévoit 50µL de calcéine diluée à 1µM par puits, soit 5ml par plaque 96 puits. On prépare ensuite une solution de lyse qui est de l'eau contenant 0,25M de sucrose (Sigma), et 0,1M EDTA (Sigma).

Pour réaliser le test, on ajoute 50µL de PBS dans les puits contrôle, et dans tous les autres puits, on ajoute 50µL de solution de marquage. Pour chaque puits, on veille à bien homogénéiser le milieu dans le puits.

On laisse incuber 1 heure. Puis, on élimine le surnageant, et on rince les cellules par ajout de 200µL de milieu de culture (4°C). on répète ce lavage 2 fois, puis on vide les puits.

On ajoute 150 µL de solution de tampon de lyse à l'ensemble des puits de la plaque.

Sur un spectrophotomètre à fluorescence Perkin Elmer, Enspire® Multimode Reader, on mesure la rétention de la calcéine par une lecture de la fluorescence de la fluorescéine (λ_{excitation} : 494nm, λ_{émission}, 512nm).

Pour chaque puits, on calcule le pourcentage d'activité MDR pour chacun des actifs testés:
■ f₀ étant le niveau basal, valeur mesurée pour les puits contenant les cellules témoins (calcéine, mais non traitées par un actif),
■ f_{Ax} étant le ratio mesuré pour chacun des autres puits (cellules traitées avec les x actifs),
alors le pourcentage d'activité MDR est : f_{Ax} x 100 /f₀

Les résultats (% de rétention de calcéine) sont les suivants :

| | |
|---|---|
| Blanc : pas de signal | 1,7% |
| Témoin (cellules non traitées) | 100,0% |
| KGF 25ng/mL | 92,3% |
| FGF 5nM | 91,8% |
| Vérapamil à 30µg/mL | 139,1% |
| Solution S₂ selon l'exemple 1 de l'invention | 79,8% |
| Diamond Sirt à 0.015% | 87,5% |
| Fraction protéique de soja hydrolysée à 1% | 84,2% |

Le produit Diamond Sirt est distribué par Akott France des laboratoires Infinitec, et se compose de microsphères de dioxide Ti couvertes d'agrégats de diamants et greffées par un peptide biomimétique de Sirt-1.

La fraction protéique de soja hydrolysée est vendue par la société Ashland sous le nom de Dynachondrine.

Si le pourcentage obtenu est inférieur à 100, l'actif stimule l'activité des MDR. Dans le cas où le pourcentage obtenu est supérieur à 100, l'actif inhibe l'activité des MDR.

On peut donc inhiber le signal, ce qui traduit une amélioration de l'activité des protéines MDR, avec des facteurs de croissance (KGF ou FGF) mais surtout avec :
* La solution de l'exemple 1, mélange d'extrait d'arbre à soie, de peptide protecteur d'ADN, et d'extrait de fleurs de grenade;
* Un peptide biomimétique de Sirtuine.

Une fraction d'extrait de soja agissant sur les mitochondries qui possède une action multiple, en particulier sur la sirtuine3, la chaîne respiratoire, les radicaux libres, la synthèse de l'ATP ...)

### Exemple 3 : Crème de jour

| Phase | Ingrédients | % |
|---|---|---|
| A | Eau Osmose | QSP 100 |
| A | Glycérine | 3 |
| A | Conservateur | QSP |
| B | Acrylates /C10-30 Alkyl acrylate crosspolymer | 0,5 |
| C | Xanthan gum | 0,1 |
| D | Cetearyl olivate and sorbitan olivate | 2 |
| D | Sodium stearoyl glutamate | 0,1 |
| D | C10-18 Triglycerides | 3 |
| D | Cetearyl alcohol | 2 |
| D | Diméthyl isosorbide | 2 |
| D | Octyldodecyl myristate | 3 |
| D | Butyrospermum parkii buttuer | 2 |
| D | Cococ caprylate | 3 |
| E | Tromethamine A 20 % | QS pH 6.5 |
| F | Huile d'Argan | 2 |
| F | Huile de Marula | 2 |
| G | Gel de Hyaluronate à 1% | 2 |
| H | Albizia julibrissin bark extract | 0,5 |
| H | Tripeptide-9 citrulline | 2 |
| I | Eau osmosée | 3 |
| I | Punica granatum flower | 1 |
| J | Saccharide isomerat | 2 |
| K | Parfum | 1 |
| K | Tocopheryl acetate | 0,1 |

Dans une cuve, on chauffe au bain-marie la phase D jusqu'à 70°-75°C. On vérifie qu'elle est bien homogène.

On prélève les quantités d'eau osmosée de la phase I. Dans la cuve de fabrication, cuve ouverte, on introduit la quantité d'eau osmosée à 70°-75°C, et on y disperse les différents ingrédients de la phase A. On agite avec la turbine jusqu'à complète solubilisation. Puis, cuve ouverte, on incorpore la phase B en mouillant bien la poudre avec la spatule. On agite sous turbine jusqu'à complète homogénéisation. Cuve ouverte, on incorpore alors la phase C en mouillant bien la poudre avec la spatule. On agite sous turbine et planétaire. Cuve ouverte, on ajoute la phase D dans la cuve de fabrication. On agite sous turbine et planétaire pendant 10 minutes. Puis on commence le refroidissement sous agitation turbine et planétaire.

### Préparation de la phase I :

Dans la quantité d'eau osmosée refroidie indiquée, ajouter les différents ingrédients de la phase I sous agitation turbine.

Agiter jusqu'à obtention d'une phase homogène et lisse. A 50°C, ajouter la phase E sous agitation turbine puis planétaire pendant 10 minutes. A 35°C, ajouter successivement les ingrédients de la phase F sous agitation turbine puis planétaire. Ajouter la phase G sous agitation turbine puis planétaire. Ajouter successivement les ingrédients de la phase H sous agitation turbine puis planétaire. Incorporer la phase I sous agitation turbine puis mettre le planétaire en marche. Ajouter la phase J sous agitation turbine puis mettre le planétaire. Ajouter successivement les ingrédients de la phase K sous agitation turbine puis planétaire. Poursuivre l'agitation turbine et planétaire jusqu'à refroidissement à 25°C.

### Exemple 4 : Sérum

| **Phase** | **Ingrédients** | **%** |
|---|---|---|
| A | Eau osmosée | QSP 100 |
| A | Conservateur | QSP |
| A | Glycérine | 3 |
| A | Propanediol | 4 |
| B | Xanthan gum | 0,2 |
| C | Sodium acryloyldimethyltaurate/VP crosspolymer | 0,2 |
| D | Cetearetg=h-30 | 0,2 |
| D | Cyclopentasiloxane and cyclohexasiloxane | 5 |
| E | Diméthyl sosorbide | 3 |
| E | Dioscorea villosa root extract | 0,1 |
| F | Albizia julibrissin bark extract | 4 |
| F | Tripeptide-9 citrulline | 2 |
| G | Gel de hyaluronate à 1% | 5 |
| H | Eau osmosée | 5 |
| H | Punica granatum flower | 1 |
| I | Saccharide isomerate | 1 |
| J | Tocopheryl acetate | 0,1 |
| J | Parfum | 0,3 |
| K | Tromethamine à 20% | QS pH 5,5 |

Dans une cuve, chauffer au bain-marie la phase D jusqu'à 70°-75°C. Vérifier qu'elle soit bien homogène. Prélever la quantité d'eau osmosée de la phase H. Dans la cuve de fabrication, cuve ouverte, introduire la quantité d'eau osmosée à 70°-75°C, et disperser les différents ingrédients de la phase A. Agiter avec la turbine jusqu'à complète solubilisation. Cuve ouverte, incorporer la phase B dans la phase A en mouillant bien la poudre avec la spatule. Agiter sous turbine jusqu'à complète homogénéisation. Cuve ouverte, incorporer la phase C en mouillant bien la poudre avec la spatule. Agiter sous turbine. Cuve ouverte, ajouter la phase D dans la cuve de fabrication. Agiter sous turbine et planétaire moyen pendant 10 minutes. Commencer le refroidissement sous agitation turbine et planétaire.

### Préparation de la phase H :

Dans la quantité d'eau osmosée refroidie indiquée, ajouter les différents ingrédients de la phase H sous agitation. Agiter jusqu'à obtention d'une phase homogène et lisse. A 50°C, ajouter la phase E sous agitation turbine puis planétaire pendant 10 minutes. A 35°C, ajouter successivement les ingrédients de la phase F sous agitation turbine puis planétaire.

Toujours sous agitation turbine puis planétaire, ajouter la phase G, puis la phase H et la phase I, et enfin successivement les ingrédients de la phase J, et la phase K. Poursuivre l'agitation turbine et planétaire jusqu'à la température de 25°C.

## Revendications

1. Composition comprenant, dans un milieu physiologiquement acceptable, au moins un extrait d'arbre à soie, un extrait de grenadier et un peptide de séquence SEQ ID N°1.

2. Composition selon la revendication 1, dans laquelle l'extrait d'arbre à soie est un extrait d'écorce d'arbre à soie.

3. Composition selon la revendication 1 ou 2, dans laquelle l'extrait d'arbre à soie est un extrait hydroalcoolique.

4. Composition selon l'une quelconque des revendications 1 à 3, dans laquelle l'extrait de grenadier est un extrait de fleur de grenadier.

5. Composition selon la revendication 4, dans laquelle l'extrait de fleur de grenadier est un extrait hydroalcoolique.

6. Composition selon l'une quelconque des revendications 1 à 5, dans laquelle la concentration finale d'extrait sec d'arbre à soie est de 0.1% à 10% en poids de la composition totale.

7. Composition selon l'une quelconque des revendications 1 à 6, dans laquelle la concentration finale d'extrait sec de fleur de grenadier est de 0.1% à 10% en poids de la composition totale.

8. Composition selon l'une quelconque des revendications 1 à 7, dans laquelle la concentration de peptide de séquence SEQ ID N°1 est de 0.01% à 10% en poids de la composition totale.

9. Composition selon l'une quelconque des revendications 1 à 8, **caractérisée par le fait qu'**elle comprend une phase aqueuse.

10. Composition selon l'une quelconque des revendications 1 à 9, **caractérisée par le fait qu'**elle comprend de l'eau en une teneur allant de 1 à 99% en poids par rapport au poids total de la composition.

11. Composition selon la revendication 10, **caractérisée par le fait qu'**elle comprend de l'eau en une teneur allant de 40 à 75 % en poids par rapport au poids total de la composition.

12. Composition selon l'une quelconque des revendications 1 à 11, **caractérisée par le fait qu'**elle se présente sous la forme d'émulsion huile-dans-eau, de gel, de crème, ou sous la forme d'un stick.

13. Procédé cosmétique pour lutter contre le vieillissement et/ou le flétrissement de la peau, comprenant l'application topique sur la peau d'une composition selon l'une quelconque des revendications 1 à 12.
